# EUROPEAN PATENT SPECIFICATION

(11) **EP 2 331 010 B1**
(45) Date of publication and mention of the grant of the patent: **23.09.2020**
(21) Application number: 09792062.3
(22) Date of filing: 28.08.2009
(51) Int. Cl.: A61F 2/95

(54) **VENA CAVA FILTER HAVING PLURALITY OF HOOKS**
HOHLVENENFILTER MIT MEHREREN HAKEN
FILTRE POUR VEINE CAVE MUNI D'UNE PLURALITÉ DE CROCHETS

(30) Priority: 29.08.2008 US 92765 P
(43) Date of publication of application: 15.06.2011
(73) Proprietor: Cook Medical Technologies LLC, Bloomington, IN 47404 (US)
(72) Inventor: BATES, Brian, L., Bloomington, IN 47401 (US)
(74) Representative: Mathys & Squire
(86) International application number: PCT/US2009/055381
(87) International publication number: WO 2010/025379

(56) References cited:
- WO-A-2007/079409
- WO-A1-2005/102212
- US-A- 6 126 673
- US-A1- 2005 277 977

## Description

### BACKGROUND OF THE INVENTION

The present invention relates to medical devices. More particularly, the invention relates to a removable vena cava clot filter that can be percutaneously placed in and removed from the vena cava of a patient.

Filtering devices that are percutaneously placed in the vena cava have been available for over thirty years. A need for filtering devices arises in trauma patients, orthopedic surgery patients, neurosurgery patients, or in patients having medical conditions requiring bed rest or non-movement. During such medical conditions, the need for filtering devices arises due to the likelihood of thrombosis in the peripheral vasculature of patients wherein thrombi break away from the vessel wall, risking downstream embolism or embolization. For example, depending on the size, such thrombi pose a serious risk of pulmonary embolism wherein blood clots migrate from the peripheral vasculature through the heart and into the lungs.

A filtering device can be deployed in the vena cava of a patient when, for example, anticoagulant therapy is contraindicated or has failed. Typically, filtering devices are permanent implants, each of which remains implanted in the patient for life, even though the condition or medical problem that required the device has passed. In more recent years, filters have been used or considered in preoperative patients and in patients predisposed to thrombosis which places the patient at risk for pulmonary embolism.

The benefits of a vena cava filter have been well established, but improvements may be made. For example, manufacturers of medical devices have been challenged in preventing or lessening implants from perforating vessel walls. As perforation of vessel walls are undesireable, there have been needs for an effective vena cava filter having features that lessen the likelihood of perforation and that can be removed after the underlying medical condition has passed.

Reference is directed to US 6126673 in which a vascular filter comprises an emboli-caturing portion having a set of helical filter-wires joined at a central region and extending in a given direction along the blood vessel in a diverging relationship to the axis of the filter, the wires terminating in free ends constructed to engage the walls of said vessel. A major mid-portion of the length of the free ended wires are of generally helical form, cooperatively related to form an effective emboli capturing array. Anchoring is accomplished by a separate assembly formed of struts and anchoring devices. A parallelogram supporting strut assembly and other members for providing linear engagement with the wall of the vena cava are shown. The parallelogram structure is shown to have filter capability by itself as well. Multiple pointed projections can be utilized to maintain contact with the vena cava wall in various discrete vessels. Document WO2005102212 shows a retrievable self-centering vena cava filter where the anchoring barb is designed such that it can be pulled out of the tissue without damaging it.

### SUMMARY

Embodiments of the present invention generally provide a removable vena cava filter having features that prevent or lessen perforation of a body vessel when implanted The invention is defined by claim 1.

In one embodiment, the present invention provides a removable filter for capturing thrombi in a body vessel. The filter comprises a plurality of primary struts comprising proximal and distal portions. Each proximal portion has a first end, wherein the first ends are attached together along a longitudinal axis. Each primary strut extends arcuately along the longitudinal axis and linearly radially. The distal portions of the primary struts are configured to expand in the body vessel, engaging the distal hooks with the body vessel. Each distal portion integrally extends from the proximal portion to a plurality of distal hooks. The distal hooks are substantially equal in size relative to each other.

In this embodiment, the filter further comprises a plurality of secondary struts having connected ends attached to each other along the center point. Each secondary strut extends arcuately along a longitudinal center plane and linearly along a diametric center plane from the connected end to a free end to centralize the filter in the expanded state in the body vessel.

In another embodiment, the filter further comprises a hub that axially houses the primary strut first ends and secondary strut first ends and a retrieval hook that extends from the hub opposite the plurality of primary struts for removal of the filter from the body vessel.

Further aspects, features, and advantages of the invention will become apparent from consideration of the following description and the appended claims when taken in connection with the accompanying drawings.

### BRIEF DESCRIPTION OF THE DRAWINGS

Figure 1 is an illustration of the anatomy of the renal veins, the iliac veins, and the vena cava in which one embodiment of a vena cava filter of the present invention is deployed;
Figure 2a is a side perspective view of one embodiment of the vena cava filter in an expanded state;
Figure 2b is a side view of a primary strut of the filter in Figure 3a in accordance with one embodiment of the present invention;
Figure 2c is a side view of the vena cava filter of Figure 3a in a collapsed state and disposed in an introducer tube;
Figure 3 is a cross-sectional view of a hub of the filter in Figure 3 taken along line 3-3;
Figure 4a is a cross-sectional view of the vena cava depicting the filter partially deployed leading with the removal hook;
Figure 4b is a cross-sectional view of the vena cava depicting the filter partially deployed leading with the distal hooks;
Figure 5 is a cross-sectional view of the vena cava in which the filter of Figure 3a has been deployed;
Figure 6a is a cross-sectional view of the vena cava of Figure 7a taken along line 8-8;
Figure 6b is a cross-sectional view of the vena cava of Figure 7a taken along line 8-8 depicting another embodiment of the filter;
Figure 7a is a cross-sectional view of a body vessel in which a retrieval sheath engages primary struts of the filter in Figure 3 for removal; and
Figure 7b is a cross-sectional view of a body vessel in which the retrieval sheath includes the filter in the collapsed state for removal.

### DETAILED DESCRIPTION OF THE INVENTION

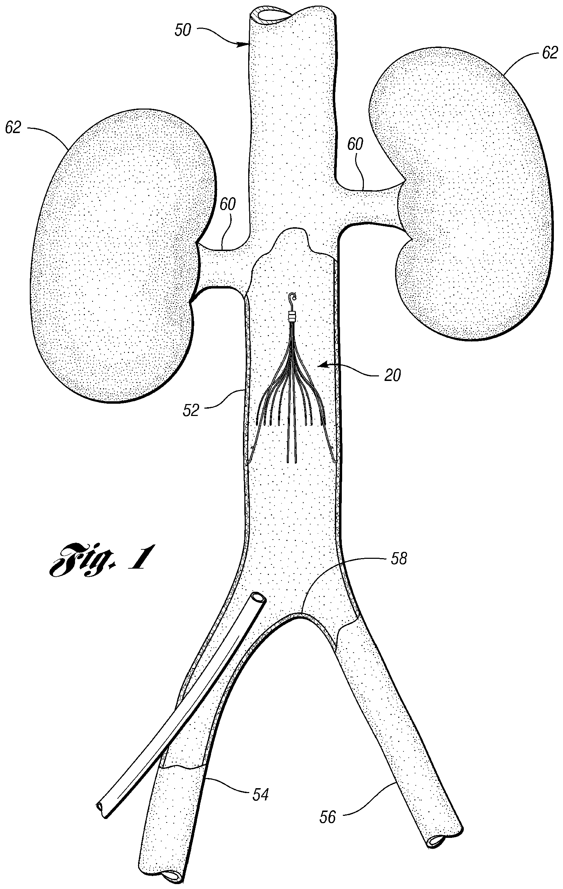

In accordance with one embodiment of the present invention, Figure 1 illustrates a vena cava filter 10 implanted in the vena cava 50. As shown, the filter 10 has features that lessen the likelihood of perforation of the vena cava wall and that can be removed after the underlying medical condition has passed. The filter 10 captures thrombi carried by the blood flowing through the iliac veins 54, 56 toward the heart and into the pulmonary arteries. As shown, the iliac veins merge at juncture 58 into the vena cava 50. The renal veins 60 from the kidneys 62 join the vena cava 50 downstream of juncture 58. The portion of the vena cava 50, between the juncture 58 and the renal veins 60, defines the inferior vena cava 52 in which the vena cava filter 10 has been percutaneously deployed through one of the femoral veins. Preferably, the vena cava filter 10 has a length smaller than the length of the inferior vena cava 52.

Figure 1a illustrates a filter 10 in an expanded state and comprising four primary struts 12 each having first ends that emanate from a hub 11. Hub 11 attaches by crimping first ends 14 of primary struts 12 together at a center point A in a compact bundle along a central or longitudinal axis X of the filter. The hub 11 has a minimal diameter for the size of wire used to form the struts.

Preferably, the primary struts 12 are formed of a superelastic material, stainless steel wire, Nitinol, cobalt-chromium-nickel-molybdenum-iron alloy, or cobalt chrome-alloy or any other suitable superelastic material that will result in a self-opening or self-expanding filter. In this embodiment, the primary struts 12 are preferably formed from wire having a round cross-section with a diameter of at least about 0.015 inches. Of course, it is not necessary that the primary struts have a round or near round cross-section. For example, the primary struts 12 could take on any shape with rounded edges to maintain non-turbulent blood flow therethrough.

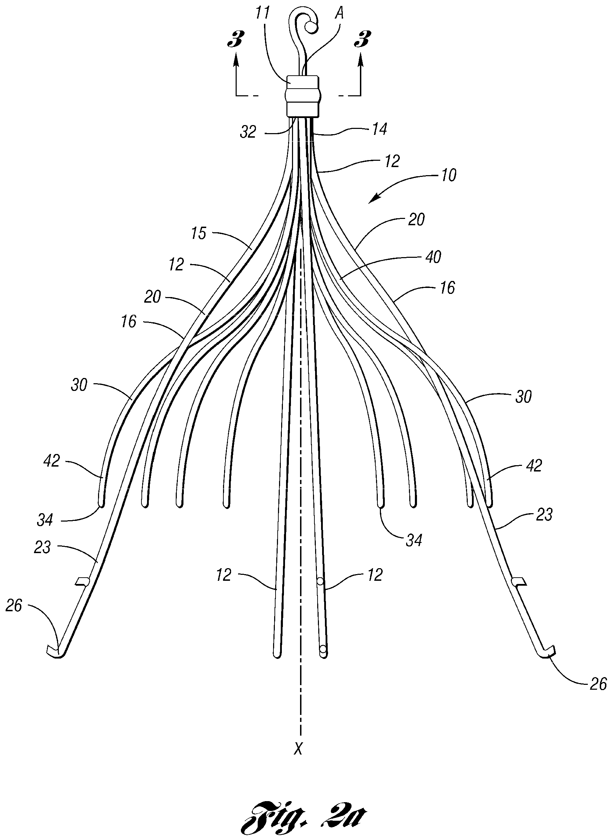

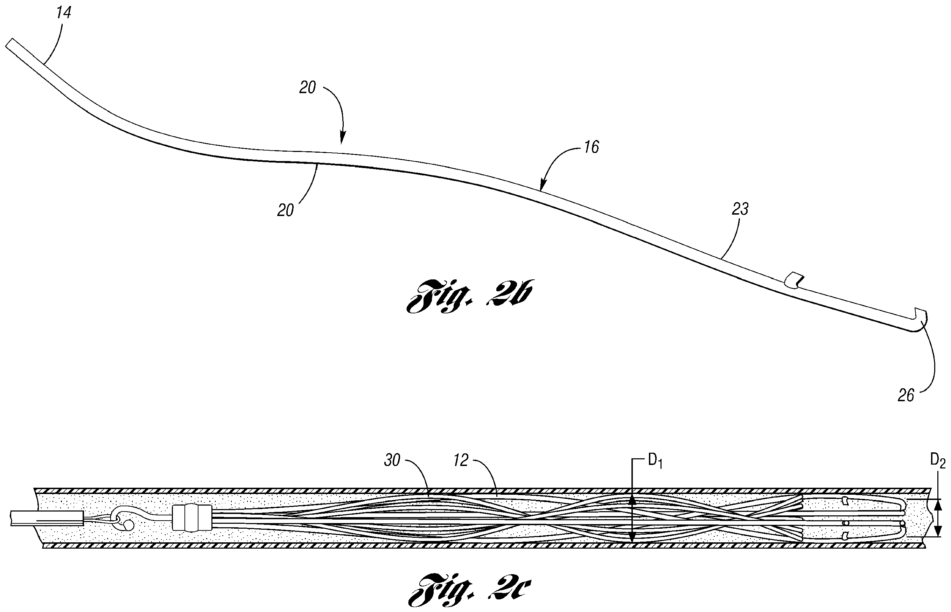

As shown in Figures 2a and 2b, each primary strut 12 includes an arcuate segment 16 having a soft S-shape. Each arcuate segment 16 is formed with a proximal portion 20 that is configured to softly bend away from the longitudinal or central axis X of the filter 10 and a distal portion 23 that is configured to softly bend toward the longitudinal axis of the filter 10. The distal portion 23 integrally extends from the proximal portion 20 to a plurality of distal hooks 26, 27 as described in greater detail below. Due to the soft bends of each arcuate segment 16, a prominence or a point of inflection on the primary strut 12 is substantially avoided to aid in non-traumatically engaging the vessel wall.

As shown in Figure 2b, each distal portion 23 of each primary strut 12 comprises a plurality of distal hooks, at least one of which will anchor to the vessel wall when the filter 10 is deployed at a delivery location in the body vessel. In this embodiment, each distal portion comprises two opposed distal hooks 26, 27 that distally extend from the distal portion and are longitudinally aligned with each other. As shown, distal hook 26 terminates at the end of primary strut 12 and distal hook 27 extends from strut 12 proximal to hook 26. Moreover, distal hooks 26 and 27 are in opposed relationship with each other as the hooks extend from the strut 12 at opposing directions as depicted in Figure 2a. It is understood that the distal portion may comprise more than two distal hooks.

The distal hooks 26, 27 are configured to avoid or lessen perforation of the vessel wall by having more surface area contact with the vessel wall than a single distal hook would otherwise have. As shown in Figures 2a and 2b, the opposed configuration of the plurality of hooks 26, 27 substantially limits or lessens perforation of the vessel wall to a length substantially equal to that of the distal hook 26. In one example, this is due to the surface area contact to the vessel wall by the opposing distal hook 27 as it would serve to resist the other hook 26 from further perforation through the vessel wall. As desired, more than two distal hooks may be used per primary strut. Moreover, the distal hooks are substantially equal in size, e.g., in length and thickness as each extends from the distal portion of the strut, relative to each other. For example, a thickness of within 0.002 inch (0.0508mm), preferably within 0.001 inch (0.0254mm), may be defined as substantially the same thickness. And, for example, a length of within 0.03 inch (0.00762mm), preferably within 0.02 inch, may be defined as substantially the same length (0.508mm).

Preferably, the thicknesses of each of the distal hooks are substantially the same as the thickness of respective primary strut. It is also preferred that the distal hooks of each primary strut are substantially the same in size, e.g., length and thickness. However, it is understood that each set of plurality of distal hooks of a particular primary strut may differ in size from another primary strut. Thus, each primary strut may comprise distal hooks that differ in size from distal hooks of another primary strut.

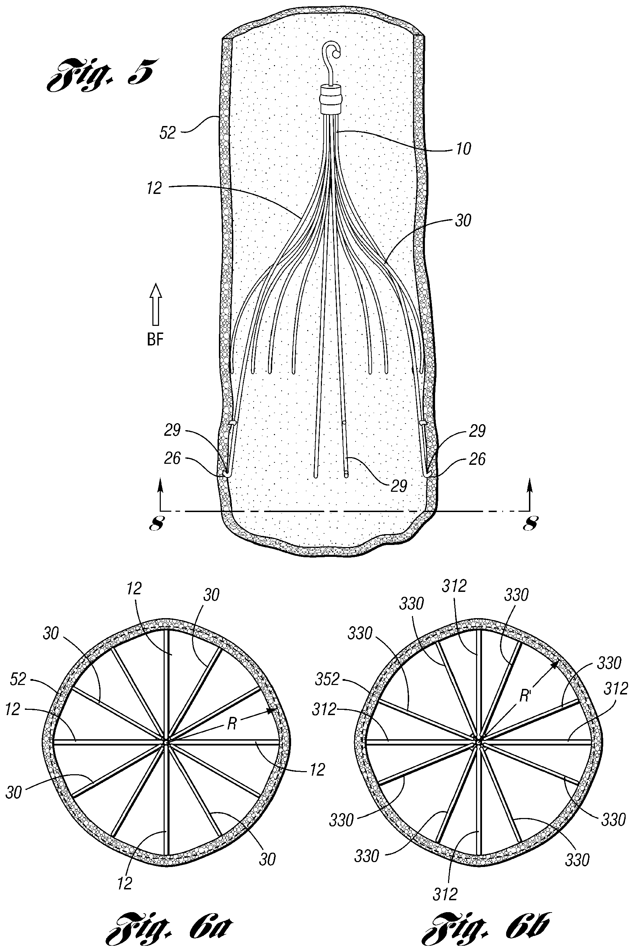

The primary struts 12 are configured to move between an expanded state for engaging the distal hooks 26, 27 with the body vessel and a collapsed state for filter retrieval or delivery. In the expanded state, each arcuate segment 16 extends arcuately along a longitudinal axis X (as shown in Figure 2a) and linearly relative to a radial axis R (as shown in Figure 6a) from the first end 14 to the distal hooks26. As shown in Figure 6a, the primary struts 12 radially extend from the first ends 14, defining the radial axis R. In this embodiment, the primary struts 12 extend linearly relative to the radial axis R and avoid entanglement with other struts.

As discussed in greater detail below, the soft bends of each arcuate segment 16 allow each primary strut 12 to cross another primary strut 12 along the longitudinal axis X in the collapsed state such that each distal hook 26 faces toward the longitudinal axis X for filter retrieval or delivery.

When the filter 10 is deployed in a body vessel, the distal hooks 26, 27 engage the walls of the body vessel to define a first axial portion to secure the filter in the body vessel. The distal hooks 26, 27 prevent the filter 10 from migrating from the delivery location in the body vessel where it has been deposited. The primary struts 12 are shaped and dimensioned such that, when the filter 10 is freely expanded, the filter 10 has a diameter of between about 25 mm and 45 mm and a length of between about 3 cm and 7 cm. For example, the filter 10 may have a diameter of about 35 mm and a length of about 5 cm. The primary struts 12 have sufficient spring strength that when the filter is deployed the distal hooks 26, 27 will anchor into the vessel wall.

In this embodiment, the filter 10 includes a plurality of secondary struts 30 having connected ends 32 that also emanate from hub 11 as shown in Figure 2a. Hub 11 attaches by crimping the connected ends 32 at the center point A of the secondary struts 30 together with the primary struts 12. In this embodiment, each primary strut 12 has two secondary struts 30 in side-by-side relationship with the primary strut 12. The secondary struts 30 extend from the connected ends 32 to free ends 34 to centralize the filter 10 in the expanded state in the body vessel. As shown, each secondary strut 30 extends arcuately along the longitudinal axis and linearly relative to the radial axis from the connected end 32 to the free end 34 for engaging the distal hooks 26, 27 with the body vessel. As with the primary struts 12, the secondary struts 30 extend linearly relative to the radial axis and avoid entanglement with other struts.

The secondary struts 30 may be made from the same type of material as the primary struts 12. However, the secondary struts 30 may have a smaller diameter, e.g., at least about 0.012 inches, than the primary struts 12. In this embodiment, each of the secondary struts 30 is formed of a first arc 40 and a second arc 42. The first arc 40 extends from the connected end 32 away from the longitudinal axis X. The second arc 42 extends from the first arc 40 towards the longitudinal axis X. As shown, two secondary struts 30 are located on each side of one primary strut 12 to form a part of a netting configuration of the filter 10. The hub 11 is preferably made of the same material as the primary struts and secondary struts to minimize the possibility of galvanic corrosion or molecular changes in the material due to welding.

When freely expanded, free ends 34 of the secondary struts 30 will expand radially outwardly to a diameter of about 25 mm to 45 mm. For example, the secondary struts 30 may expand radially outwardly to a diameter of between about 35 mm and 45 mm. The second arcs 42 of the free ends 34 engage the wall of a body vessel to define a second axial portion where the vessel wall is engaged. The secondary struts 30 function to stabilize the position of the filter 10 about the center of the body vessel in which it is deployed.

As a result, the filter 10 has two layers or portions of struts longitudinally engaging the vessel wall of the body vessel. The length of the filter 10 is preferably defined by the length of a primary strut 12. Furthermore, the diameter of the hub 11 is defined by the size of a bundle containing the primary struts 12 and secondary struts 30. In this embodiment, the eight secondary struts 30 minimally add to the diameter of the hub 11 or the overall length of the filter 10, due to the reduced diameter of each secondary strut 30. This is accomplished while maintaining the filter 10 in a centered attitude relative to the vessel wall and formed as a part of the netting configuration of the filter 10. As shown, removal hook 46 extends from hub 11 opposite primary and secondary struts 12 and 30.

In this embodiment, each arcuate segment 16 has a thickness of at least about 0.015 inch (0.381 mm) and a tensile strength of between about 285,000 pounds per square inch (psi) (1.965GPa) and 330,000 psi (2.275GPa). Each distal hook 26 is integral with the arcuate segment 16 and has the thickness and the tensile strength of the arcuate segment. Each secondary strut 30 has a thickness of at least about 0.012 inch (0.3048mm) and a tensile strength of between about 285,000 psi (1.965GPa) and 330,000 psi (2.275GPa).

Figure 2c illustrates the filter 10 in a collapsed state disposed in a delivery/retrieval tube 94 for delivery or retrieval. As shown, the filter 10 is shaped for each primary strut 12 to cross another primary strut 12 along the longitudinal axis X. As a result, in the collapsed state, the distal hooks 26, 27 are configured to invert or inwardly face the longitudinal axis X for retrieval and delivery of the filter 10. This inverted or inwardly facing configuration of the distal hooks 26, 27 allows for simplified delivery and retrieval of filter 10. For example, a concern that the distal hooks 26, 27 may scrape, scratch, or tear the inner wall of a delivery/retrieval tube is eliminated, since the filter 10 of the present invention is shaped to have the distal hooks 26, 27 face each other in the collapsed state. Merely one delivery/retrieval tube with a loop snare mechanism may be used to deliver or retrieve the filter 10 of the present invention.

Moreover, in the collapsed state, each primary strut 12 is configured to cross another primary strut 12 along the longitudinal axis X such that the arcuate segments 16, proximal portions 20 or distal portions 23, occupy a first diameter D₁. In this embodiment, the first diameter is greater than a second diameter D₂ occupied by the distal hooks 26, 27 for filter retrieval or delivery. It has been found that the first diameter of the arcuate segments 16 serves to clear a path of retrieval, reducing radial force from the sheath or body vessel on the distal hooks 26, 27 during removal of the filter 10 from a patient. Reducing the radial force on the distal hooks 26, 27 assists in preventing the distal hooks 26, 27 from scraping, scratching, or tearing the inner wall of a sheath during removal of the filter 10 from a patient.

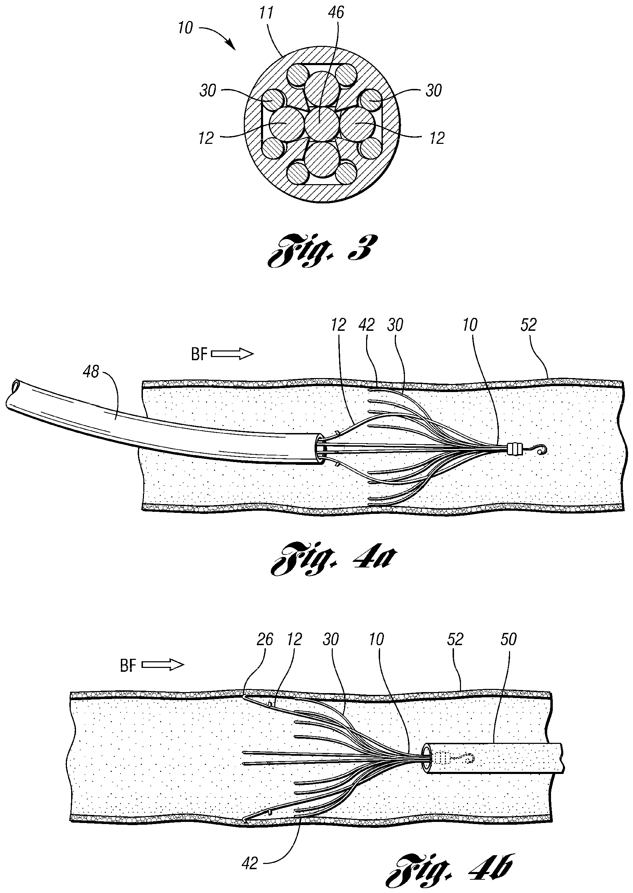

Figure 3 illustrates a cross-sectional view of the filter 10 of Figure 2a at hub 11. As shown, the hub 11 houses a bundle of first ends 14 of the four primary struts 14 and connected ends 32 of secondary struts 30. Figure 3 further depicts the configurations of the primary and secondary struts 12 and 30. In this embodiment, the primary struts 12 are spaced between two secondary struts 30. Of course, the primary struts 12 may be spaced between any other suitably desired number of secondary struts 30 without falling beyond the scope or spirit of the present invention.

In this embodiment, Figures 4a and 4b both illustrate the filter 10 partially deployed in inferior vena cava 52. Figure 4a shows the filter 10 being delivered by a delivery tube 48 through the vasculature of a patient and Figure 4b shows the filter 10 being delivered by a delivery tube 50 through the jugular vein of a patient. For deployment of the filter 10, a delivery tube is percutaneously inserted through the patient's vessel such that the distal end of the delivery tube is at the location of deployment. In this embodiment, a wire guide is preferably used to guide the delivery tube to the location of deployment. In Figure 4a, the filter 10 is inserted through the proximal end of the delivery tube 48 with the removal hook 46 leading and distal hooks 26, 27 of the primary struts 12 held by a filter retainer member for delivery via the femoral vein of a patient.

In Figure 4b, the filter 10 is inserted through the proximal end of the delivery tube 50 with the distal hooks 26, 27 of the primary struts 12 leading and the removal hook 46 trailing for delivery via the jugular vein of a patient. In this embodiment, a pusher wire having a pusher member at its distal end may be fed through the proximal end of the delivery tube 50 thereby pushing the filter 10 until the filter 10 reaches the distal end of the delivery tube 50 to a desired location.

During deployment, the secondary struts 30 expand first to centralize or balance the filter within the vessel. When the free ends of the secondary struts emerge from the distal end of either of the delivery tubes 48 or 50, the secondary struts 30 expand to an expanded position as shown in both Figures 4a and 4b. The second arcs 42 engage the inner wall of the vessel. The second arcs 42 of the secondary struts 30 function to stabilize the attitude of filter 10 about the center of the body vessel. When delivering through the jugular vein (Figure 4b), the filter 10 is then pushed further by the pusher wire (not shown) until it is fully deployed.

When the filter 10 is fully expanded in the vena cava, the distal hooks 26, 27 of the primary struts 12 and the second arcs 42 of the secondary struts 30 are in engagement with the vessel wall. The distal hooks 26, 27 of the primary struts 12 have anchored the filter 10 at the location of deployment in the vessel, preventing the filter 10 from moving with the blood flow through the vessel. As a result, the filter 10 is supported by two sets of struts that are spaced axially along the length of the filter.

Figures 2a and 5 illustrate the filter 10 fully expanded after being deployed in inferior vena cava 52. As shown, the inferior vena cava 52 has been broken away so that the filter 10 can be seen. The direction of the blood flow BF is indicated in Figure 5 by the arrow that is labeled BF. The distal hooks 26, 27 at the ends of the primary struts 12 are shown as being anchored in the inner lining of the inferior vena cava 52. The distal hooks 26, 27 function to retain the filter 10 in the location of deployment.

The spring biased configuration of the primary struts 12 further causes the distal hooks 26, 27 to engage the vessel wall and anchor the filter at the location of deployment. After initial deployment, the pressure of the blood flow on the filter 10 contributes in maintaining the hooks 26, 27 anchored in the inner lining of the inferior vena cava 52. As seen in Figures 2a and 5, the second arcs 42 of secondary struts 30 also have a spring biased configuration to engage with the vessel wall.

As seen in Figures 2a and 5, the hub 11 and removal hook 46 are positioned downstream from the location at which the distal hooks 26, 27 are anchored in the vessel. When captured by the struts 12 and 30, thrombi remains lodged in the filter. The filter 10 along with the thrombi may then be percutaneously removed from the vena cava. When the filter 10 is to be removed, the removal hook 46 is preferably grasped by a retrieval instrument that is percutaneously introduced in the vena cava in the direction of removal hook 16 first.

Figure 6a depicts a netting configuration or pattern formed by the primary struts 12, secondary struts 30, and the hub 11 relative to radial axis R. The netting pattern shown in Figure 6a functions to catch thrombi carried in the blood stream prior to reaching the heart and lungs to prevent the possibility of a pulmonary embolism. The netting pattern is sized to catch and stop thrombi that are of a size that are undesirable to be carried in the vasculature of the patient. Due to its compacted size, the hub minimally resists blood flow.

Figure 6a depicts the netting pattern including primary struts and secondary struts at substantially equal angular space relative to each other. The netting pattern provides an even distribution between the primary and secondary struts to the blood flow, increasing the likelihood of capturing thrombi. However, as shown in Figure 6b, it is to be understood that each of the sets of primary struts 312 and secondary struts 330 may be independently spaced substantially equally at their respective portions relative to radial axis R'. For example, the secondary struts 330 may be spaced equally relative to the other secondary struts 330 and the primary struts 312 may be spaced equally relative to the other primary struts 312. As a result, the netting pattern in this embodiment shown by the cross-sectional view of the vena cava (taken along line 8-8) will have uneven or unequal spacing between the primary struts 312 and secondary struts 330.

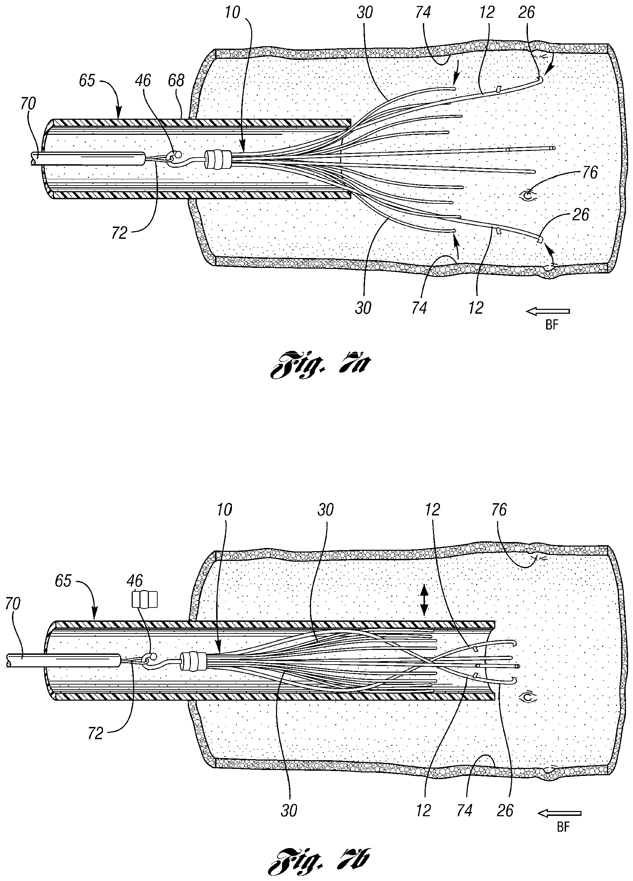

Figure 7a illustrates part of a retrieval device 65 being used in a procedure for removing the filter 10 from the inferior vena cava 52. In this example, the retrieval device 65 is percutaneously introduced into the superior vena cava via the jugular vein. In this procedure, a removal catheter or sheath 68 of the retrieval device 65 is inserted into the superior vena cava. A wire 70 having a loop snare 72 at its distal end is threaded through the removal sheath 68 and is exited through the distal end of the sheath 68. The wire 70 is then manipulated by any suitable means from the proximal end of the retrieval device such that the loop snare 72 captures the removal hook 46 of the filter 10. Using counter traction by pulling the wire 70 while pushing the sheath 68, the sheath 68 is passed over the filter 10.

As the sheath 68 passes over the filter 10, the primary struts 12 and then the secondary struts 30 engage the edge of the sheath 68 and are caused to pivot or undergo bend deflection at the hub 11 toward the longitudinal axis of the filter. The pivoting toward the longitudinal axis causes the ends of the struts 12 and 30 to be retracted from the vessel wall. In this way, only surface lesions 74 and small point lesions 76 on the vessel wall are created in the removal procedure. As shown, the surface lesions 74 are created by the ends of the secondary struts 30 and the small point legions 76 are created by the distal hooks 26, 27 of the primary struts 12. However, it is to be noted that any other suitable procedure may be implemented to remove the filter from the patient.

Although the embodiments of this device have been disclosed as being constructed from wire having a round cross section, it could also be cut from a tube of suitable material by laser cutting, electrical discharge machining or any other suitable process.

The primary and secondary struts can be formed from any suitable material that will result in a self-opening or self-expanding filter, such as shape memory alloys. Shape memory alloys have the desirable property of becoming rigid, that is, returning to a remembered state, when heated above a transition temperature. A shape memory alloy suitable for the present invention is Ni-Ti available under the more commonly known name Nitinol. When this material is heated above the transition temperature, the material undergoes a phase transformation from martensite to austenic, such that material returns to its remembered state. The transition temperature is dependent on the relative proportions of the alloying elements Ni and Ti and the optional inclusion of alloying additives.

In other embodiments, both the primary struts and the secondary struts are made from Nitinol with a transition temperature that is slightly below normal body temperature of humans, which is about 98.6°F (310.15K). Thus, when the filter is deployed in the vena cave and exposed to normal body temperature, the alloy of the struts will transform to austenite, that is, the remembered state, which for the present invention is an expanded configuration when the filter is deployed in the body vessel. To remove the filter, the filter is cooled to transform the material to martensite which is more ductile than austenite, making the struts more malleable. As such, the filter can be more easily collapsed and pulled into the sheath for removal.

In other embodiments, both the primary struts and the secondary struts 40 are made from Nitinol with a transition temperature that is above normal body temperature of humans, which is about 98.6° F (310.15K). Thus, when the filter is deployed in the vena cave and exposed to normal body temperature, the struts are in the martensitic state so that the struts are sufficiently ductile to bend or form into a desired shape, which for the present invention is an expanded configuration. To remove the filter, the filter is heated to transform the alloy to austenite so that the filter becomes rigid and returns to a remembered state, which for the filter is a collapsed configuration.

While the present invention has been described in terms of preferred embodiments, it will be understood, of course, that the invention is not limited thereto since modifications may be made to those skilled in the art, particularly in light of the foregoing teachings.

## Claims

1. A removable filter (10) for capturing thrombi in a body vessel, the filter comprising:
a plurality of primary struts (12) comprising proximal (20)and distal (23) portions, each proximal portion having a first end (14), the first ends attached together along a longitudinal axis, each primary strut extending arcuately along the longitudinal axis and linearly radially, each distal portion integrally extending from the proximal portion to a distal hook (26, 27), the distal portions of the primary struts being configured to expand in the body vessel to engage the distal hooks with the body vessel; and
a plurality of secondary struts (30) having connected ends attached to each other along the center point, each secondary strut extending arcuately along a longitudinal center plane and linearly along a diametric center plane from the connected end to a free end to centralize the filter in the expanded state in the body vessel.
**characterised in** each distal portion integrally extending from the proximal portion to a plurality of distal hooks (26, 27), the distal hooks being substantially equal in size relative to each other.

2. The removable filter of claim 1 further comprising:
a hub configured to axially house the first ends of the plurality of primary struts; and
a retrieval hook extending from the hub opposite the plurality of primary struts for removal of the filter from the body vessel.

3. The removable filter of claim 1 wherein the proximal portion is configured to extend radially from the longitudinal axis of the filter and the distal portion is configured to extend radially toward the longitudinal axis of the filter.

4. The removable filter of claim 1 wherein each primary strut is formed of a superelastic material, stainless steel wire, Nitinol, cobalt-chromium-nickel-molybdenum-iron alloy, or cobalt-chrome alloy.

5. The removable filter of claim 2 wherein each secondary strut is formed of a superelastic material, stainless steel wire, Nitinol, cobalt-chromium-nickel-molybdenum-iron alloy, or cobalt-chrome alloy.

6. The removable filter of Claim 1, further comprising:
a hub axially housing the primary strut first ends and secondary strut first ends; and
a retrieval hook extending from the hub opposite the plurality of primary struts for removal of the filter from the body vessel.

7. The removable filter of claim 6 wherein the proximal portion is configured to extend radially from the longitudinal axis of the filter and the distal portion is configured to extend radially toward the longitudinal axis of the filter.

8. The removable filter of claim 6 wherein each primary strut is formed of a superelastic material, stainless steel wire, Nitinol, cobalt-chromium-nickel-molybdenum-iron alloy, or cobalt chrome-alloy.

9. The removable filter of claim 6 wherein each secondary strut is formed of a superelastic material, stainless steel wire, Nitinol, cobalt-chromium-nickel-molybdenum-iron alloy, or cobalt chrome-alloy.

## Patentansprüche

1. Entfernbarer Filter (10) zum Einfangen von Thromben in einem Körpergefäß, wobei der Filter umfasst:
eine Vielzahl von Primärstreben (12), die proximale (20) und distale (23) Anteile umfassen, wobei jeder proximale Anteil ein erstes Ende (14) aufweist, die ersten Enden entlang einer Längsachse aneinander befestigt sind, jede Primärstrebe sich im Bogen entlang der Längsachse und linear radial erstreckt, jeder distale Anteil sich integral von dem proximalen Anteil zu einem distalen Haken (26, 27) erstreckt, wobei die distalen Anteile der Primärstreben so ausgestaltet sind, dass sie in das Körpergefäß expandieren, um die distalen Haken in Eingriff mit dem Körpergefäß zu bringen; und
eine Vielzahl von Sekundärstreben (30) mit verbundenen Enden, die entlang des Mittelpunkts aneinander befestigt sind, wobei jede Sekundärstrebe sich im Bogen entlang einer längsgerichteten Mittelebene und linear entlang einer diametrischen Mittelebene von dem verbundenen Ende bis zu einem freien Ende erstreckt, um den Filter in dem expandierten Zustand in dem Körpergefäß zu zentralisieren,
**dadurch gekennzeichnet, dass** jeder distale Anteil sich integral von dem proximalen Anteil zu einer Vielzahl distaler Haken (26, 27) erstreckt, wobei die distalen Haken eine relativ gleiche Größe aufweisen.

2. Entfernbarer Filter nach Anspruch 1, ferner umfassend:
einen Ansatz, der ausgestaltet ist, um die ersten Enden der Vielzahl von Primärstreben aufzunehmen; und
einen Rückholhaken, der sich von dem Ansatz gegenüber der Vielzahl von Primärstreben erstreckt, um den Filter aus dem Körpergefäß zu entfernen.

3. Entfernbarer Filter nach Anspruch 1, wobei der proximale Anteil ausgestaltet ist, um sich radial von der Längsachse des Filters zu erstrecken, und der distale Anteil ausgestaltet ist, um sich radial in Richtung der Längsachse des Filters zu erstrecken.

4. Entfernbarer Filter nach Anspruch 1, wobei jede Primärstrebe aus einem superelastischen Material, rostfreiem Stahldraht, Nitinol, Kobalt-Chrom-Nickel-Molybdän-Eisen-Legierung oder Kobalt-Chrom-Legierung gebildet ist.

5. Entfernbarer Filter nach Anspruch 2, wobei jede Sekundärstrebe aus einem superelastischen Material, rostfreiem Stahldraht, Nitinol, Kobalt-Chrom-Nickel-Molybdän-Eisen-Legierung oder Kobalt-Chrom-Legierung gebildet ist.

6. Entfernbarer Filter nach Anspruch 1, ferner umfassend:
einen Ansatz, der die ersten Enden der Primärstrebe und die ersten Enden der Sekundärstrebe aufnimmt; und
einen Rückholhaken, der sich von dem Ansatz gegenüber der Vielzahl von Primärstreben erstreckt, um den Filter aus dem Körpergefäß zu entfernen.

7. Entfernbarer Filter nach Anspruch 6, wobei der proximale Anteil ausgestaltet ist, um sich radial von der Längsachse des Filters zu erstrecken, und der distale Anteil ausgestaltet ist, um sich radial in Richtung der Längsachse des Filters zu erstrecken.

8. Entfernbarer Filter nach Anspruch 6, wobei jede Primärstrebe aus einem superelastischen Material, rostfreiem Stahldraht, Nitinol, Kobalt-Chrom-Nickel-Molybdän-Eisen-Legierung oder Kobalt-Chrom-Legierung gebildet ist.

9. Entfernbarer Filter nach Anspruch 6, wobei jede Sekundärstrebe aus einem superelastischen Material, rostfreiem Stahldraht, Nitinol, Kobalt-Chrom-Nickel-Molybdän-Eisen-Legierung oder Kobalt-Chrom-Legierung gebildet ist.

## Revendications

1. Filtre amovible (10) pour capturer des thrombi dans un vaisseau corporel comprenant :
une pluralité d'entretoises primaires (12) comprenant des portions proximales (20) et
distales (23), chaque portion proximale ayant une première extrémité (14), les premières extrémités étant attachées les unes aux autres le long d'un axe longitudinal, chaque entretoise primaire s'étendant sous forme arquée le long de l'axe longitudinal et linéairement radialement, chaque portion distale s'étendant intégralement depuis la portion proximale jusqu'à un crochet distal (26, 27), les portions distales des entretoises primaires étant configurées pour se déployer dans le vaisseau corporel de manière à mettre en prise les crochets distaux avec le vaisseau corporel ; et
une pluralité d'entretoises secondaires (30) ayant des extrémités connectées attachées les unes aux autres le long du point central, chaque entretoise secondaire s'étendant sous forme arquée le long d'un plan central longitudinal et linéairement le long d'un plan central diamétral depuis l'extrémité connectée jusqu'à une extrémité libre de manière à centrer le filtre dans l'état déployé dans le vaisseau corporel ;
**caractérisé en ce que** chaque portion distale s'étend intégralement depuis la portion proximale jusqu'à une pluralité de crochets distaux (26, 27), les dimensions des crochets distaux étant substantiellement identiques les unes aux autres.

2. Filtre amovible selon la revendication 1, comprenant en outre :
un moyeu configuré pour recevoir axialement les premières extrémités de la pluralité d'entretoises primaires ; et
un crochet de récupération s'étendant depuis le moyeu à l'opposé de la pluralité d'entretoises primaires pour retirer le filtre du vaisseau corporel.

3. Filtre amovible selon la revendication 1, dans lequel la portion proximale est configurée pour s'étendre radialement depuis l'axe longitudinal du filtre et la portion distale est configurée pour s'étendre radialement vers l'axe longitudinal du filtre.

4. Filtre amovible selon la revendication 1, dans lequel chaque entretoise primaire est formée d'un matériau superélastique, d'un fil en acier inoxydable, de nitinol, d'un alliage de cobalt-chrome-nickel-molybdène-fer, ou d'un alliage de cobalt-chrome.

5. Filtre amovible selon la revendication 2, dans lequel chaque entretoise secondaire est formée d'un matériau superélastique, d'un fil en acier inoxydable, de nitinol, d'un alliage de cobalt-chrome-nickel-molybdène-fer, ou d'un alliage de cobalt-chrome.

6. Filtre amovible selon la revendication 1, comprenant en outre :
un moyeu recevant axialement les premières extrémités des entretoises primaires et les premières extrémités des entretoises secondaires ; et
un crochet de récupération s'étendant depuis le moyeu à l'opposé de la pluralité d'entretoises primaires pour retirer le filtre du vaisseau corporel.

7. Filtre amovible selon la revendication 6, dans lequel la portion proximale est configurée pour s'étendre radialement depuis l'axe longitudinal du filtre et la portion distale est configurée pour s'étendre radialement vers l'axe longitudinal du filtre.

8. Filtre amovible selon la revendication 6, dans lequel chaque entretoise primaire est formée d'un matériau superélastique, d'un fil en acier inoxydable, de nitinol, d'un alliage de cobalt-chrome-nickel-molybdène-fer, ou d'un alliage de cobalt-chrome.

9. Filtre amovible selon la revendication 6, dans lequel chaque entretoise secondaire est formée d'un matériau superélastique, d'un fil en acier inoxydable, de nitinol, d'un alliage de cobalt-chrome-nickel-molybdène-fer, ou d'un alliage de cobalt-chrome.
